# EUROPÄISCHE PATENTSCHRIFT

(11) **EP 0 956 824 B1**
(45) Veröffentlichungstag und Bekanntmachung des Hinweises auf die Patenterteilung: **17.03.2004**
(21) Anmeldenummer: 99107504.5
(22) Anmeldetag: 14.04.1999
(51) Int. Cl.: A61B 17/00

(54) **Instrument bestehend aus einem in den Markraum einzusetzenden Schaft und einem Griffteil**
Instrument comprising a shaft for insertion into the medullary canal and a handle
Instrument pour l'insertion dans le canal médullaire comportant une tige et une poignée

(30) Priorität: 28.04.1998 DE 29807671 U
(43) Veröffentlichungstag der Anmeldung: 17.11.1999
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 380 309
- DE-U- 9 407 621
- US-A- 5 089 003
- US-A- 5 443 471

## Beschreibung

Bevor der Schaft einer Hüftprothese in den Markraum des proximalen Oberschenkelknochens eingesetzt werden kann, muß nach der Resektion des Kopfhalses der Markraum geöffnet und entsprechend der Form des einzusetzenden Schafts ausgeräumt und geformt werden. Dies geschieht mit einer Raspel, deren Schaft der Gestalt des Prothesenschafts gleicht. Das obere Ende der Raspel entspricht der Resektionsebene. Darauf erhebt sich ein Schaft, dessen Richtung mit derjenigen des Kopfhalses übereinstimmt, damit ein Raspelwerkzeug zur Endbearbeitung der Resektionsfläche aufgesetzt werden kann (EP-B 166 085) oder ein Probegelenkkopf aufgesetzt werden kann. Ähnliches gilt für einen Versuchsschaft. Häufig dient die Raspel gleichzeitig als Versuchsschaft.

Es ist bekannt (DE-U 9407621, US-A-4990149, EP-A-380309, US-A-5089003), zum Einsetzen und Lösen des Schafts einen Griffteil vorzusehen, der über Kupplungsteile lösbar mit dem Schaft verbunden werden kann. Er verläuft im wesentlichen etwa parallel zur Richtung des Schafts , damit Hammerschläge auf sein als Amboß ausgebildetes Ende zum Eintreiben oder Herausziehen des Schafts etwa in Längsrichtung des Schafts auf diesen wirken.

Die Kupplungsteile zum Verbinden des Schafts mit dem Griffteil umfassen einerseits den weiter oben beschriebenen Zapfen am oberen Ende des Schafts und andererseits am Griffteil eine diesen Zapfen aufnehmende Bohrung. Da der Zapfen in Kopf-Hals-Richtung verläuft, ragt er schräg seitlich vom Schaft (bezogen auf dessen mittlere Längsrichtung) von diesem weg. Damit das untere Ende des Griffteils den Zapfen aufnehmen kann, ist es im allgemeinen gegenüber der sonstigen Richtung des Griffteils ebenfalls entsprechend abgewinkelt. Daraus ergibt sich, daß der Griffteil überwiegend ein wenig seitlich versetzt gegenüber dem Schaft verläuft, wenn auch im wesentlichen parallel zur Längsrichtung des Schafts.

Die bei den bekannten Instrumenten dieser Art vorgesehenen Kupplungsteile umfassen Mittel zum Arretieren des Zapfens in der Bohrung, um die Verbindung zwischen Schaft und Griffteil für die Benutzung zu sichern. Diese Arretiermittel sind lösbar, damit der Griffteil von dem Zapfen abgenommen werden kann. Die bekannten Arretiermittel lassen zu wünschen übrig. Wenn sie einen federbelasteten Riegel aufweisen, der in eine keilförmige Nut des Zapfens eingreift (EP-A-0 380 309, US-A-5089003) kann der Riegel gegen die Federkraft ausweichen; die Arretierung ist unsicher. Bei einem anderen bekannten Instrument (DE-C 196 31 984) müssen zum Kuppeln des Schafts mit dem Griffteil S-förmige Eingriffsglieder dieser beiden Teile miteinander in Eingriff gebracht werden, deren Zusammenwirken durch eine darüberzuschiebende Hülse gesichert wird. Das hat den Nachteil, daß die Kupplungs- und Arretierungsmittel mit Mühe in der Tiefe der Wunde befestigt werden müssen. Bei einer wieder anderen Kupplungseinrichtung (Prospekt "Das PCA-Hüft-Total-System" der Firma Howmedica Kiel) ist zwar ein an den Kupplungseinrichtungen angreifender Hebel außerhalb der Operationswunde bewegbar; jedoch ist die Hebelbewegung sehr ausladend, was unter den beengten Platzverhältnissen des Operationsfeldes nachteilig ist. Schließlich haben die meisten bekannten Instrumente der geschilderten Art den Nachteil, daß ihre Kupplungseinrichtungen kompliziert sind und sie deshalb schwer zu reinigen sind.

Der Erfindung liegt die Aufgabe zugrunde, ein Instrument der geschilderten und im Oberbegriff des Anspruchs 1 angegebenen Art im Hinblick auf sichere Funktion, einfache Bedienbarkeit außerhalb des unmittelbaren Wundbereichs und/oder leichte Reinigung zu verbessern. Dies gelingt durch die Merkmale des Anspruchs 1, dessen Oberbegriff auf dem Instrument von EP-A-380309 basiert ist.

Der in Längsrichtung des Griffteils beweglich geführte Stab ist an seinem oberen Ende mit einer Handhabe verbunden. Diese kann am schaftfernen Ende des Griffteils vorgesehen sein und liegt damit während der Operation leicht zugänglich fern von der Wunde. Der Stab greift mit seinem schaftnahen Ende in der Arretierstellung durch eine Bohrung des Zapfens hindurch. Daher können auch dann, wenn zwischen dem Griffteil und dem Schaft hohe Kräfte wirken, keine den Stab aus der Arretierstellung herausdrängenden Kräfte verursacht werden. Der Stab ist beiderseits der im Zapfen vorgesehenen Bohrung durch die im Griffteil vorgesehene Führungsbohrung abgestützt. Er kann daher hohe Kräfte aufnehmen, die sicher auf den Körper des Griffteils übertragen werden. Zur Sicherung des Stabs in der Arretierstellung ist an der Handhabe eine Rasteinrichtung vorgesehen.

Zweckmäßigerweise wird der Stab in einer unten offenen Bohrung des Kupplungsteils geführt, um zu Reinigungszwecken leicht herausgenommen werden zu können. Zu diesem Zweck wird er von seiner Handhabe gelöst, beispielsweise abgeschraubt. Oberhalb des Kupplungsteils liegt er zweckmäßigerweise offen, um dadurch die Reinigung zu erleichtern. Er kann von einer zur Seite des Griffteils hin offenen Nut aufgenommen sein, die im wesentlichen seinen gesamten Querschnitt aufnimmt, damit der Griffteil ohne Rücksicht auf den Stab gefaßt und benutzt werden kann.

Die Erfindung wird im folgenden näher unter Bezugnahme auf die Zeichnung erläutert, die ein vorteilhaftes Ausführungsbeispiel veranschaulicht. Es zeigen:
- Fig. 1: eine Gesamtdarstellung in verkleinertem Maßstab und
- Fig. 2: die wesentlichen Teile in etwa natürlichem Maßstab.

Die Raspel 1 ist verbunden mit einem Griffteil 2. Die Raspel 1 umfaßt einen Schaft 9, der in den Markkanal des proximalen Oberschenkelknochens einzusetzen ist. Er endet oben in einer ebenen Fläche 3, die schräg zur Längsrichtung des Schafts 9 steht. Von dieser Fläche 3 erhebt sich etwa lotrecht und also ebenfalls schräg im Verhältnis zur Längsrichtung des Schafts 9 ein Zapfen 4.

Der Griffteil 2 besteht aus einem langgestreckten Griff 5, der zur Längsrichtung des Raspelschafts 9 etwa parallel verläuft und diesem gegenüber ein wenig seitlich versetzt ist. Er läuft unten in einen Kupplungsteil 6 aus, der entsprechend der Schrägungsrichtung des Zapfens 4 schräg gegenüber dem Griff 5 des Griffteils und dem Schaft 9 der Raspel 1 verläuft. Der Winkel zwischen dem Zapfen 4 und dem Kupplungsteil 6 einerseits und der Längsrichtung des Raspelschafts 9 und des Griff 5 des Griffteils 2 andererseits entspricht dem CCD-Winkel der zugehörigen Prothese und liegt daher in derselben Größenordnung wie übliche CCD-Winkel. Der Kupplungsteil 6 enthält eine Bohrung 7, die den Zapfen 4 passend aufnimmt. Der Durchmesser der Bohrung 7 ist wenig größer als derjenige des Zapfens 4, so daß sich dieser leicht in die Bohrung einsetzen läßt, aber dennoch eine gute Kupplungsführung gegeben ist.

Am oberen Ende trägt der Griffteil 2 einen Amboß 8, der ober- und unterseitig Schlagflächen bildet zum Ein- und Ausschlagen des Raspelschafts 9 aus dem Knochen.

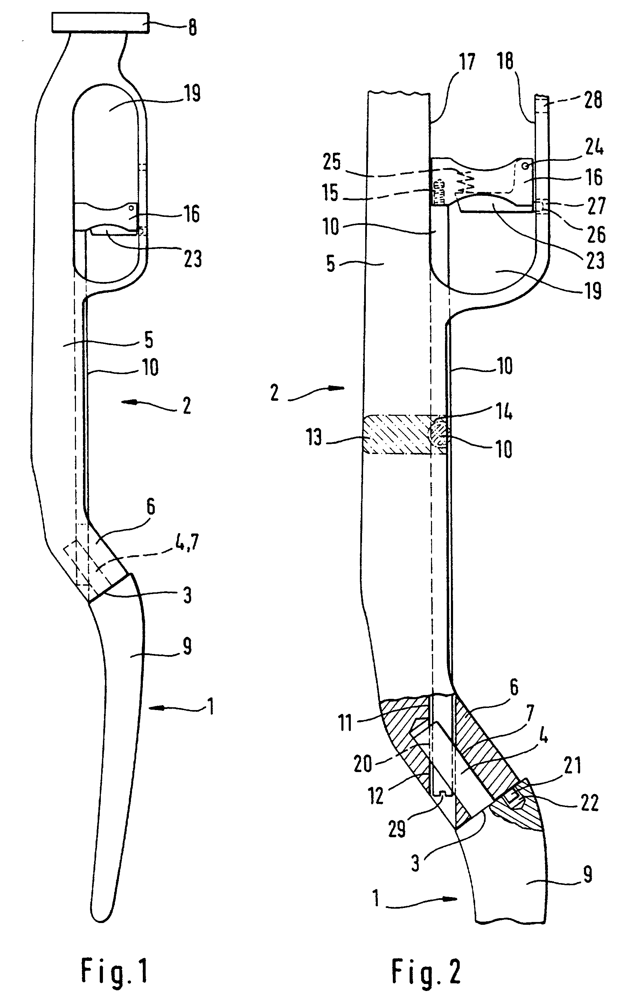

Zum Arretieren des Zapfens 4 in der Bohrung 7 dient der Stab 10, der im Kupplungsteil 6 des Griffteils 2 in fluchtenden Bohrungen 11, 12 oberhalb und unterhalb der Bohrung 7 geführt ist. Oberhalb des Kupplungsteils 6 im Bereich des Griffs 5 liegt er, wie bei 13 durch Querschnittsdarstellung angedeutet, in einer nach außen offenen Nut 14 des Griffs. Sein oberes Ende ist bei 15 eingeschraubt in einen Schieber 16, der zwischen den Flanken 17,18 einer Grifföffnung 19 in Längsrichtung des Griffs 5 geführt ist. Der Schieber 16 bildet die Handhabe zur Längsverschiebung des Stabs 10. Der Zapfen 4 der Raspel 1 enthält eine im eingesetzten Zustand mit den Bohrungen 11,12 fluchtende Zapfenbohrung 20. In der in Fig. 2 dargestellten Stellung ragt das untere Ende des Stabs 10 durch diese Bohrung 20 und arretiert ihn dadurch in der Bohrung 7. Er ist in der Lage, beträchtliche Kupplungskräfte aufzunehmen, da er oberhalb und unterhalb des Zapfens 4 in den Bohrungen 11,12 geführt ist. Dabei ist der Zapfen 4 auch gegen Drehung gesichert, so daß Kräfte nicht nur in Längsrichtung, sondern auch rotativ vom Griffteil 2 auf den Schaft 9 der Raspel 1 übertragen werden können. Dennoch kann es zweckmäßig sein, zusätzlich eine Drehsicherung vorzusehen, die in Fig. 2 angedeutet ist als Vorsprung 21 am unteren Ende des Kupplungsteils 6, der eingreift in eine Sackbohrung 22 in der Fläche 3 der Raspel 1. Der Vorsprung 21 und die Bohrung 22 sind exzentrisch zu den Zapfen 4 bzw. der Bohrung 7 gelegen.

Will man die in Fig. 2 geschlossen dargestellte Kupplung lösen, so zieht man den Stab 10 mittels des Schiebers 16 nach oben, bis er die Bohrung 20 des Zapfens 4 verlassen hat. Der Griffteil 2 kann dann von der Raspel 1 leicht abgenommen werden.

Damit der Schieber 16 nicht ungewollt die Arretierstellung bzw. die gelöste Stellung verlassen kann, ist er mit einer Rasteinrichtung versehen. An dem Schieber 16 ist ein Auslöser 23 um den Schwenkpunkt 24 gelagert und durch eine Feder 25 im Gegenuhrzeigersinn beaufschlagt, so daß sein in der Zeichnung rechts gelegener Rücken sich an der Flanke 18 der Grifföse 19 anlegt. Er trägt dort einen Vorsprung 26, der mit einer Rastöffnung 27 zusammenwirkt, die die Arretierstellung bezeichnet. Gewünschtenfalls kann noch eine weitere Rastöffnung 28 vorgesehen werden, die der gelösten Stellung zugeordnet ist. Will der Arzt die Kupplung lösen, so braucht er lediglich mit dem Finger unterhalb des Schiebers 16 durch die Grifföse zu greifen und den Finger nach oben zu ziehen, wobei der Auslöser 23 gegen die Federkraft in den Schieber 16 gedrückt wird, bis der Vorsprung 26 die Rastöffnung 27 verläßt und der Schieber 16 samt dem Stab 10 nach oben gleiten kann. Zum Schließen der Kupplung verfährt man umgekehrt.

Die Bohrung 12 ist nach unten hin offen. Der Stab 10 ist in seiner Stirnfläche mit einem Schraubenzieherschlitz 29 oder dergleichen versehen, um den Ansatz eines Werkzeugs zu ermöglichen, mit dessen Hilfe seine Verschraubung 15 mit dem Schieber 16 gelöst wird. Der Stab 10 und der Schieber 16 können dann leicht zu Reinigungszwecken von dem Griffteil 2 getrennt werden.

## Patentansprüche

1. Chirurgisches Instrument bestehend aus einem in den Markraum des proximalen Oberschenkelknochens einzusetzenden Schaft (9) und einem Griffteil (2), der etwa parallel zur Richtung des Schafts (9) mit dessen oberen Ende mittels beiderseitiger Kupplungsteile lösbar verbindbar ist, die einen vom oberen Schaftende (3) schräg zur Seite vorragenden Zapfen (4) und am Griffteil (2) eine den Zapfen (4) aufnehmende Bohrung (7) mit lösbaren Mitteln zum Arretieren des Zapfens (4) in der Bohrung (7) umfassen, die von dem Ende eines in Längsrichtung des Griffteils (2) beweglich geführten Stabs (10) und einer dieses Ende aufnehmenden Ausnehmung (20) in dem Zapfen (4) gebildet sind, wobei das obere Ende des Stabs (10) mit einer Handhabe (16) verbunden ist, **dadurch gekennzeichnet, daß** die Handhabe mit einer Rasteinrichtung (23-27) zur Sicherung des Stabs in der Arretierstellung versehen ist, die Ausnehmung in dem Zapfen (4) eine durchgehende Bohrung (20) ist und der Stab (10) in seiner Arretierstellung beiderseits des Zapfens (4) in einer Führungsbohrung (11,12) gehalten ist.

2. Instrument nach Anspruch 1, **dadurch gekennzeichnet, daß** der Stab (10) oberhalb des Kupplungsteils (6) offen an der Seite des Griffteils (2) geführt ist.

## Claims

1. A surgical instrument comprising a shaft (9) to be inserted into the medullary caviity of the proximal femur and a handle part (2) which can be releasably connected approximately parallel to the direction of the shaft (9) to the upper end thereof by means of coupling members at two ends, which comprise a pin (4) projecting from the upper shaft end (3) obliquely sidewards and on the handle part (2) a bore (7) accommodating the pin (4) with releasable means for locking the pin (4) in the bore (7), which means are formed by the end of a rod (10) guided movably in the longitudinal direction of the handle part (2) and a recess (20) in the pin (4) accommodating said end, wherein the upper end of the rod (10) is connected to a trigger (16), **characterised in that** the trigger is provided with a locking mechanism (23-27) for securing the rod in the locking position, the recess in the pin (4) is a continuous bore (20) and the rod (10)) is retained in its locking position on either side of the pin (4) in a guide bore (11,12).

2. An instrument according to Claim 1, **characterised in that** the rod (10) is guided above the coupling part (6) openly on the side of the handle part (2).

## Revendications

1. Instrument chirurgical constitué d'une tige (9) à introduire dans le canal médullaire de l'os proximal du fémur, ainsi que d'une poignée (2) qui peut être reliée de manière séparable, approximativement parallèlement à la direction de la tige (9), à son extrémité supérieure, au moyen d'éléments d'accouplement des deux côtés, lesquels comprennent un tenon (4) dépassant obliquement vers le côté de l'extrémité supérieure (3) de la tige et, sur la poignée (2), un perçage (7) recevant le tenon, avec des moyens séparables pour bloquer le tenon (4) dans le perçage (7), lesquels sont formés par l'extrémité d'une barre (10) guidée déplaçable dans la direction longitudinale de la poignée (2), et d'un évidement (10), recevant cette extrémité, dans le tenon (4), l'extrémité supérieure de la barre (10) étant reliée à une manette (16), **caractérisé en ce que** la manette est pourvue d'un dispositif d'encliquetage (23-27) pour bloquer la barre dans la position de blocage, l'évidement du tenon (4) est un trou débouchant (20) et la barre (10) est maintenue dans sa position de blocage des deux côtés du tenon (4) dans un perçage de guidage (11, 12).

2. Instrument selon la revendication 1, **caractérisé en ce que** la barre (10) est guidée au-dessus de l'élément d'accouplement (6), ouverte sur le côté de la poignée (2).
